# DEMANDE DE BREVET EUROPEEN

(11) **EP 2 072 071 A1**
(43) Date de publication de la demande: **24.06.2009**
(21) Numéro de dépôt: 07123463.7
(22) Date de dépôt: 18.12.2007
(51) Int. Cl.: A61M 5/00

(54) **Couvercle de nest à seringues**

(71) Demandeur: Sybermat, 1420 Braine-l'Alleud (BE)
(72) Inventeur: Cousin, Géry, Georges, Albert, Marie, Ghislain, 1640, RHODE ST GENESE (BE)
(74) Mandataire: Gevers, François

(57) **Abrégé**

Couvercle (10) de nest (1) d'une hauteur (h) comprenant une plate-forme (11), un premier et un deuxième doigts (12, 14) présentant ladite longueur prédéterminée (h) entre une saillie (16) à l'extrémité de la plate-forme (11), chaque saillie (16) étant agencée pour passer d'une position de préhension à une position ouverte, et étant reliée à des moyens de commande (17) à cet effet.

## Description

La présente invention se rapport à un couvercle de nest à seringue.

Les nests sont des présentoirs à seringues, généralement en polypropylène. Les nests comprennent une plaque basale munie de tubes s'étendant tous dans une même direction au travers desquels les seringues sont enfilées et présentent une hauteur h. Les seringues présentent à une extrémité, opposée à l'orifice d'injection auquel sera fixée éventuellement une aiguille, une collerette d'un diamètre supérieur à celui de la seringue.

Lorsque les seringues sont enfilées dans les tubes, les collerettes reposent sur l'extrémité de ces tubes qui est libre, c'est-à-dire non reliée à la plaque basale. Cette façon de conditionner les seringues est largement répandue. Lorsqu'une société pharmaceutique souhaite conditionner un produit dans une seringue, en particulier dans une seringue en verre, elle s'adresse à des fabricants de seringues et elles sont livrées en présentoirs plus communément appelés nests. Généralement les nests sont munis d'un capot de protection qui protège les seringues de la casse, les seringues étant disposées parallèlement et maintenues substantiellement écartées l'une de l'autre justement grâce auxdits nests.

Les capots sont généralement de type boîte et présentent un rebord interne sur lequel vient reposer la plaque basale du nest.

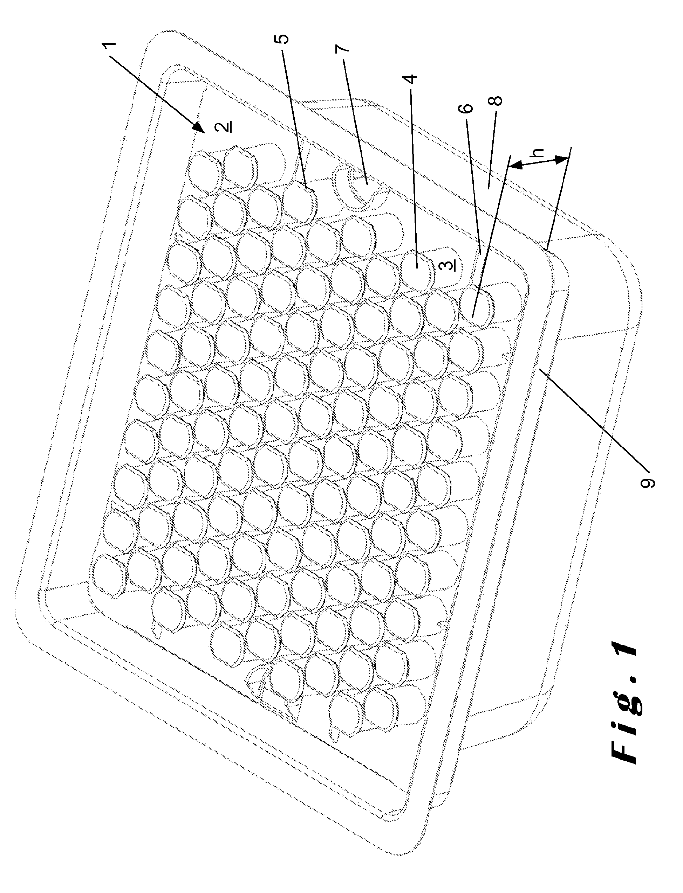

Un nest muni de son capot est illustré à la figure 1.

Les nests comprennent généralement un orifice sur deux côtés parallèles afin de pouvoir y introduire les doigts de la main pour la prise.

Les seringues sont ensuite remplies du produit qu'elles sont sensées contenir par l'orifice opposé à l'orifice d'injection défini ci-avant c'est-à-dire par celui qui est muni de la collerette.

Un bouchon est alors introduit et le poussoir du piston, quant à lui n'est ajouté qu'à la toute fin du conditionnement, juste avant l'emballage.

Généralement, il est souhaitable de réaliser une étape d'autoclavage supplémentaire afin de stériliser, avant le conditionnement final, l'extérieur de la seringue et ce y compris la partie du corps de seringue située entre le bouchon auquel le poussoir sera relié et la collerette pour éviter tout risque ultime de contamination.

Un dispositif connu comprend un peigne muni de dents. Les seringues sont enfilées entre les dents et on pince un couvercle au-dessus des collerettes reposant sur les dents du peigne, dans le but de les autoclaver tout en permettant de les maintenir dans la position inversée. En effet, le couvercle du peigne, grâce aux collerettes des seringues permet de les retourner toutes simultanément et de les autoclaver avec l'orifice de remplissage vers le bas pour éviter toute accumulation de vapeur ou d'eau dans la partie entre ledit bouchon et la collerette.

On connaît également des boîtes de manutention comme décrit dans les documents EP 1088566 et EP 1574228 qui présentent l'avantage de protéger les seringues et de pouvoir être superposées.

Malheureusement ces dispositifs nécessitent de «dénester», c'est-à-dire d'enlever du nest, les seringues, de les enfiler entre les dents des peignes ou des boîtes de manutention, pour ensuite les enlever des peignes ou boîtes et les conditionner dans leur emballage final.

II existe donc un besoin de réduire ces étapes de manipulation qui sont réalisées dans des environnements où les normes de qualité et de sécurité sont drastiques et exigeantes et où toute étape se révèle particulièrement coûteuse en validation, en temps, en appareillage, en espace, en traitement de l'environnement (surface au sol, air utilisé, etc.).

De plus, toute manipulation présente un risque de casse des seringues plus élevé une fois qu'elles sont sorties de leur nest.

Pour résoudre ce problème, il est prévu, suivant l'invention, un dispositif tel que mentionné au début, comprenant
- une plate-forme,
- un premier doigt s'étendant substantiellement perpendiculairement à ladite plate-forme et présentant une longueur prédéterminée h, le long d'une première arête de ladite plate-forme,
- un deuxième doigt s'étendant substantiellement perpendiculairement à ladite plate-forme, dans une direction similaire au premier doigt et présentant ladite longueur prédéterminée h, le long d'une deuxième arête de la plate-forme faisant face à la première arête,
- chaque doigt étant muni à une extrémité d'une saillie s'étendant substantiellement perpendiculairement audit doigt, chaque saillie étant agencée pour passer d'une position de préhension à une position ouverte, et
- des moyens de commande reliés à ladite saillie et agencés pour permettre le passage de chaque saillie de la position de préhension à la position ouverte.

Lorsque les seringues dans les nests doivent être autoclavées, elles doivent l'être avec l'orifice de remplissage vers le bas pour évacuer l'humidité ou la vapeur. Dès lors, puisque les seringues qui viennent d'être remplies reposent dans le nest, elles sont orientées dans le sens inverse, c'est-à-dire celui où la plaque basale repose sur le rebord du capot et où les tubes s'étendent vers le haut. Les collerettes des seringues sont donc en haut et l'orifice d'injection vers le bas. De cette façon, lorsque le couvercle de nest est placé sur le nest, la plate-forme repose sur les collerettes des seringues et les doigts s'étendant de la première et de la deuxième arête s'engagent dans les orifices présents sur la plaque basale du nest. Les saillies à l'extrémité des doigts peuvent alors passer sous la plaque basale du nest, c'est-à-dire passer de la position ouverte à la position de préhension sous l'action des moyens de commande.

Puisque la hauteur des tubes (collerette des seringues et plaque basale comprise) présente une valeur h identique à la longueur du doigt (entre la plate-forme et la saillie), les seringues sont parfaitement maintenues en place et peuvent être retournées en vue de leur autoclavage avec les collerettes vers le bas.

Avantageusement, lesdits doigts sont traversés par un axe relié à une extrémité à ladite saillie de manière solidaire et relié à l'autre extrémité de manière solidaire auxdits moyens de commande au travers de ladite plate-forme, lesdits moyens de commande comprenant un levier pivotant entre une position de préhension et une position ouverte dans lesquelles ladite saillie est respectivement en position de préhension et en position ouverte.

Dans cette forme de réalisation avantageuse, faire passer le levier de la position ouverte à la position de préhension fait pivoter les saillies en dessous de la plaque basale du nest.

Dans une variante, le doigt est solidaire de ladite saillie et desdits moyens de commande et le doigt pivote par rapport à ladite plate-forme d'une position dans laquelle ladite saillie est en position de préhension à une position dans laquelle ladite saillie est en position ouverte et inversement.

Dans cette variante où tout est solidaire, le risque d'encrassement et de grippage est réduit.

Particulièrement, lesdits moyens de commande sont situés du côté de ladite plate-forme qui est opposé à celui duquel lesdits doigts s'étendent, c'est-à-dire que lorsque le couvercle est posé sur les collerettes des seringues et que les doigts s'étendent vers le bas, les moyens de commande sont alors situés au-dessus de la plate-forme, ce qui les rend aisément accessibles pour faire passer les saillies de la position ouverte à la position de préhension et vice versa.

Dans une forme de réalisation avantageuse, le couvercle de nest selon l'invention comprend en outre une première paroi latérale et une deuxième paroi latérale s'étendant respectivement de ladite première et deuxième arête dans la direction desdits doigts. Ceci renforce significativement le couvercle selon l'invention en protégeant les doigts et en rigidifiant l'ensemble. En outre, la première et la deuxième paroi latérale favorisent le bon positionnement du couvercle sur ledit nest.

Dans une autre forme de réalisation avantageuse, le couvercle comprend en outre une troisième et une quatrième parois latérales, parallèles l'une à l'autre et s'étendant d'une troisième et d'une quatrième arête de ladite plate-forme, dans la direction desdits doigts. Dès lors, si le nest est rectangulaire le couvercle peut avantageusement comporter quatre parois latérales définissant une enceinte rectangulaire dans laquelle le nest est logé. De cette façon, la sécurité est maximale et la préhension optimale. La prise est parfaitement stable et le risque de casse de seringues négligeable.

De préférence, ladite plate-forme est munie d'orifices qui permettent l'écoulement de la vapeur ou de tout autre liquide qui aurait pénétré dans le corps de la seringue. En outre, les orifices permettent que le fluide de stérilisation puisse pénétrer partout dans le corps des seringues, entre les seringues pour parfaitement stériliser ce qui a été appelé l'extérieur des seringues.

Dans une autre forme de réalisation avantageuse, lesdites parois latérales présentent des encoches. Ceci permet également une meilleure stérilisation des seringues en facilitant l'accès du fluide de stérilisation mais permet également une meilleure prise en jouant le rôle de poignée.

De préférence, ledit couvercle est en un matériau résistant à une stérilisation chimique ou par autoclavage et est de préférence de l'acier inoxydable.

Avantageusement, lesdites parois latérales présentent des moyens d'ajustement de la position dudit couvercle.

Dans une forme de réalisation telle que décrite ci-avant, les moyens d'ajustement de la position, en particulier des encoches sur les extrémités des parois latérales opposées à la plate-forme permettent de loger des membrures généralement présentes sur la plaque basale du nest. De cette façon, une fois que la positon du nest est ajustée, le couvercle ne peut plus bouger.

Dans une forme de réalisation avantageuse, chaque saillie présente au moins une face biseautée et de préférence, celle faisant face à la plate-forme, de façon à faciliter la pénétration de la saillie sous le nest, en particulier quand il repose sur le rebord dudit capot lorsque la saillie passe de la position ouverte à la position de préhension.

D'autres formes de réalisation du dispositif selon l'invention sont mentionnées dans les revendications annexées.

D'autres caractéristiques, détails et avantages de l'invention ressortiront de la description donnée ci-après, à titre non limitatif et en faisant référence aux dessins annexés.

La figure 1 est une vue en perspective d'un nest contenant 100 seringues avec son capot.

La figure 2 est une vue en perspective d'une partie du couvercle selon l'invention comprenant un premier doigt.

La figure 3 est une vue en perspective du couvercle de nest selon l'invention en cours de placement sur un nest et son capot.

La figure 4 est une vue en perspective du couvercle de nest selon l'invention en position de préhension sur un nest muni des seringues, prêt à être retourné et placé dans un autoclave.

Sur les figures, les éléments identiques ou analogues portent les mêmes références.

Comme on peut le voir à la figure 1, un nest 1 est un présentoir à seringues qui comprend une plaque basale 2 munie de tubes 3 s'étendant tous dans une direction perpendiculairement à la plaque basale 2 au travers desquels les seringues 4 sont enfilées. Les collerettes 5 des seringues 4 reposent alors sur les extrémités des tubes 3 qui sont opposées à la plaque basale 2.

Le nest 1 comprend en outre des membrures 6 de renforcement ainsi que deux orifices 7 de passage des doigts, par exemple du manutentionneur qui doit prendre ledit nest.

Le nest 1 est muni d'un capot de protection 8 qui comprend un rebord 9 sur lequel le nest 1 repose. Comme on peut le voir, les tubes présentent une hauteur prédéterminée qui, additionnée de l'épaisseur de la collerette et de celle de la plaque basale vaut h.

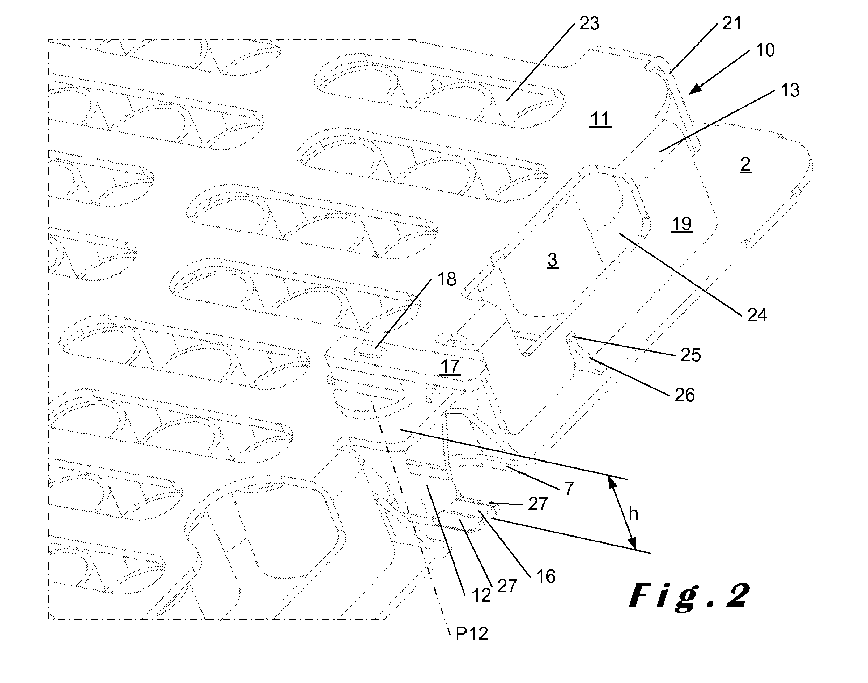

La figure 2 illustre le couvercle de nest 10 selon l'invention.

Comme on peut le voir, le couvercle 10 comprend une plate-forme 11, un premier doigt 12 qui s'étend perpendiculairement à la plate-forme 11, le long d'un axe perpendiculaire P12, le long d'une première arête 13 de la plate-forme 11. Le couvercle comprend également un deuxième doigt 14, qui s'étend perpendiculairement à la plate-forme 11, le long d'un axe perpendiculaire P14, le long d'une deuxième arête 15 de la plate-forme 11.

Chaque doigt est muni à une extrémité d'une saillie 16 qui est sensiblement perpendiculaire à l'axe du doigt. A la figure 2, la saillie 16 du doigt 12 est en position ouverte, c'est-à-dire en position de non-préhension ; la position de préhension étant celle où la saillie est positionnée sous le nest.

En outre, le couvercle 10 de nest 1 selon l'invention comprend des moyens de commande 17 sous la forme d'un levier pivotant entre une position de préhension et une position ouverte dans lesquelles ladite saillie est respectivement en position de préhension et en position ouverte.

Le levier 17 et la saillie 16 sont reliés à une extrémité chacun d'un axe 18 de section polygonale qui est solidaire en pivot du levier 17 et de la saillie 16.

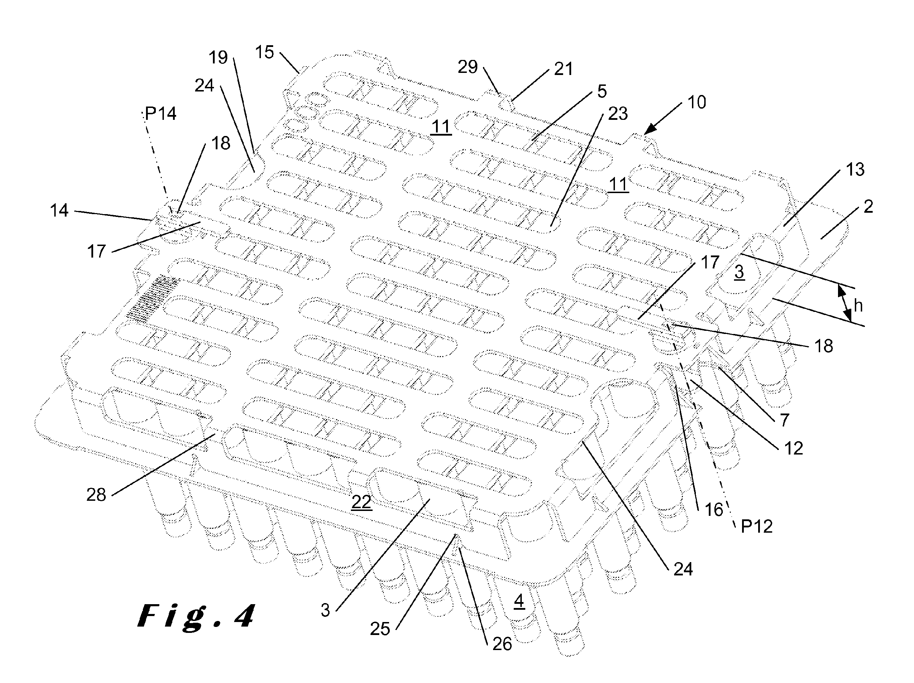

Le couvercle 10 est alors amené avec les doigts 12, 14 orientés vers le bas au-dessus du nest 1, la plate-forme 11 reposant sur les collerettes 5 des seringues 4 ou sur le haut des tubes 3 du nest 1 selon que le nest est plein ou non. Les doigts 12, 14 sont introduits dans les orifices 7 de passage des doigts du manutentionneur du nest 1 et les moyens de commande 17 et les saillies 16 sont en position ouverte. Pour que le couvercle permette de retourner le nest, il faut alors passer les moyens de commande 17 en position de préhension, ce qui aura pour effet que les saillies 16 passent en position de préhension (figure 4) et que le couvercle soit pincé solidairement au nest en exerçant une pression sur le collerettes 5 des seringues 4.

Dans une variante, chaque doigt 12, 14 est solidaire de la saillie 16 et des moyens de commande 17. Le doigt 12, 14 pivote alors simultanément avec les moyens de commande et la saillie 16 par rapport à ladite plate-forme 11 de la position de préhension à la position ouverte et vice-versa.

De façon à être les plus accessibles possible, les moyens de commande 17 sont situés du côté de ladite plate-forme 11 opposé à celui qui sera en contact avec les seringues 4 ou le nest 1 et donc celui duquel les doigts 12, 14 s'étendent.

Le couvercle selon l'invention comprend une première 19, une deuxième 20, une troisième 21 et une quatrième 22 parois latérales. Les parois latérales 19, 20, 21, 22 s'étendent des premières 13, deuxième 15, troisième 28 et quatrième 29 arêtes de la plate-forme dans la direction des doigts 12, 14.

La plate-forme 11 comprend des orifices 23. Lorsque le couvercle 10 de nest est en prise avec le nest 1 et donc que les saillies 16 et les moyens de commande 17 sont en position de préhension, le couvercle est particulièrement ajusté, et bien fixé au nest. Dès lors, les seringues qui sont dans le nest avec l'orifice de remplissage vers le haut peuvent être inversées et placées avec l'orifice de remplissage vers le bas dans l'autoclave. De cette façon la vapeur montante peut aisément pénétrer dans le corps des seringues tel que défini précédemment et lorsqu'elle est condensée elle peut aisément s'écouler vers le bas par l'orifice de remplissage.

Les orifices 23 de la plate-forme contribuent donc à la pénétration optimale du fluide de stérilisation qui peut également être un gaz de stérilisation à l'éthylène au lieu de la vapeur de l'autoclave ainsi qu'à l'écoulement de celui-ci si nécessaire.

Les orifices 23 de la plate-forme 11 sont agencés pour être alignés et coïncider avec les orifices de remplissage des seringues, les collerettes 5 desdites seringues 4 devant reposer sur la matière constituant la plate-forme 11 autour des orifices 23.

Avantageusement, les parois latérales 19, 20, 21 et 22 sont également munies d'orifices 24 également appelés encoches 24 qui ont le même rôle que les orifices 23 en plus d'éventuellement servir de poignées.

Le couvercle selon l'invention est de préférence en acier inoxydable et les parois latérales 19, 20, 21 et 22 présentent des encoches d'ajustement 25 de la position dudit couvercle par rapport à des membrures 26 généralement présentes sur le nest 1.

Chaque saillie 16 présente au moins une face biseautée 27 et de préférence deux pour faciliter la pénétration de la saillie sous le nest qui repose sur le rebord du capot.

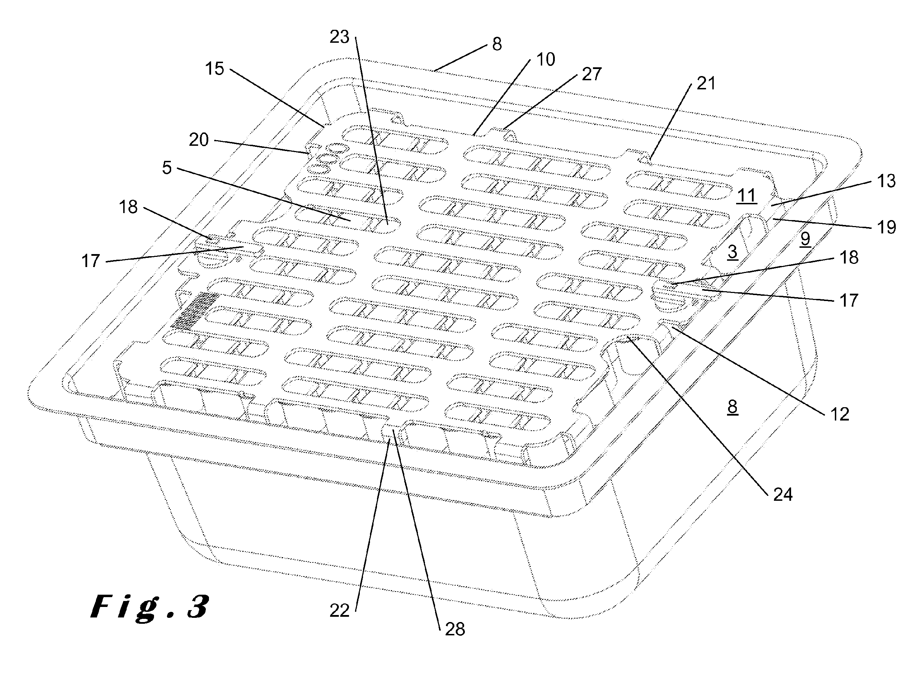

La figure 3 représente le couvercle 10 selon l'invention placé sur un nest 1 qui est rempli de seringues 4 qui repose lui-même sur le rebord 9 du capot 8. Les moyens de commande 17 du deuxième doigt 14 sont en position de préhension tandis que les moyens de commande et donc la saillie 16 aussi, sont en position ouverte.

A la figure 4, les moyens de commande 17 du premier doigt 12 ainsi que la saillie 16 ont été passés en position de préhension. Le couvercle est donc pincé sur le nest et ses seringues et le tout forme un ensemble sécurisé et sensiblement solidaire puisque la hauteur du nest à laquelle l'épaisseur de collerettes est ajoutée vaut h qui est également la hauteur entre la saillie 16 destinée à être placée sous le nest et la face de la plate-forme 11 qui est destinée à être posée sur les collerettes des seringues.

Le nest muni de son couvercle est alors ôté du capot et peut être retourné de manière sûre dans une enceinte de stérilisation et en particulier dans un autoclave.

Dans une forme particulière de l'invention, la mise du couvercle peut être automatisée. Dans ce cas, une pince peut se loger dans les encoches 24 pour soulever le nest muni de son couvercle et un moteur peut être relié aux leviers 18.

II est bien entendu que la présente invention n'est en aucune façon limitée aux formes de réalisation décrites ci-dessus et que bien des modifications peuvent y être apportées sans sortir du cadre des revendications annexées.

Par exemple, le nombre de doigts peut être bien plus élevé, on peut envisager que le couvercle comporte quatre doigts ou plus en fonction par exemple de la forme du nest.

## Revendications

1. Couvercle (10) de nest (1) d'une hauteur (h) comprenant
- une plate-forme (11),
- un premier doigt (12) s'étendant substantiellement perpendiculairement à ladite plate-forme (11) et présentant une longueur prédéterminée (h), le long d'une première arête de ladite plate-forme,
- un deuxième doigt (14) s'étendant substantiellement perpendiculairement à ladite plate-forme (11), dans une direction similaire au premier doigt et présentant ladite longueur prédéterminée (h), le long d'une deuxième arête (15) de la plate-forme (11) faisant face à la première arête (13),
- chaque doigt (12, 14) étant muni à une extrémité d'une saillie (16) s'étendant substantiellement perpendiculairement audit doigt (12, 14), chaque saillie (16) étant agencée pour passer d'une position de préhension à une position ouverte, et
- des moyens de commande (17) reliés à ladite saillie (16) et agencés pour permettre le passage de chaque saillie (16) de la position de préhension à la position ouverte.

2. Couvercle selon la revendication 1, dans lequel lesdits doigts (12, 14) sont traversés par un axe (18) relié à une extrémité à ladite saillie (16) de manière solidaire et relié à l'autre extrémité de manière solidaire auxdits moyens de commande (17) au travers de ladite plate-forme (11), lesdits moyens de commande (17) comprenant un levier pivotant entre une position de préhension et une position ouverte dans lesquelles ladite saillie (16) est respectivement en position de préhension et en position ouverte.

3. Couvercle selon la revendication 1, dans lequel ledit doigt (12, 14) est solidaire de ladite saillie (16) et desdits moyens de commande (17) et dans lequel ledit doigt (12, 14) pivote par rapport à ladite plate-forme (11) d'une position dans laquelle ladite saillie (16) est en position de préhension à une position dans laquelle ladite saillie (16) est en position ouverte et inversement.

4. Couvercle (10) selon la revendication 3, dans laquelle lesdits moyens de commande (17) sont situés du côté de ladite plate-forme (11) qui est opposé à celui duquel lesdits doigts (12, 14) s'étendent.

5. Couvercle (10) selon l'une quelconque des revendications précédentes, comprenant en outre une première paroi latérale (19) et une deuxième paroi latérale (20) s'étendant respectivement de ladite première (13) et deuxième arête (15) dans la direction desdits doigts (12, 14).

6. Couvercle (10) selon l'une quelconque des revendications précédentes, comprenant en outre une troisième (21) et une quatrième (22) parois latérales, parallèles l'une à l'autre et s'étendant d'une troisième (28) et d'une quatrième (29) arête de ladite plate-forme (11), dans la direction desdits doigts (12, 14).

7. Couvercle (10) selon l'une des revendications précédentes dans lequel ladite plate-forme (11) est munie d'orifices (23).

8. Couvercle (10) selon la revendication 5 ou la revendication 6, dans lequel chaque paroi latérale (19, 20, 21, 22) présente des encoches (24).

9. Couvercle (10) selon l'une des revendications précédentes dans lequel ledit couvercle (10) est en un matériau résistant à une stérilisation chimique ou par autoclavage et est de préférence de l'acier inoxydable.

10. Couvercle (10) selon l'une des revendications 5 ou 6 dans lequel lesdites parois latérales (19, 20, 21, 22) présentent des moyens d'ajustement de la position (25) audit couvercle (10).

11. Couvercle selon l'une quelconque des revendications précédentes, dans lequel chaque saillie (16) présente au moins une face biseautée (27) et de préférence, celle faisant face à la plate-forme.
